# EUROPEAN PATENT SPECIFICATION

(11) **EP 3 076 902 B1**
(45) Date of publication and mention of the grant of the patent: **05.02.2025**
(21) Application number: 14867162.1
(22) Date of filing: 03.12.2014
(51) Int. Cl.: A61L 27/24, A61L 27/38, A61L 27/50, A61P 19/02, A61P 43/00, A61F 2/44

(54) **METHOD OF REPAIRING AN ANNULUS AND COLLAGEN GEL COMPOSITION**
VERFAHREN ZUR REPARATUR EINES ANNULUS UND KOLLAGENGELZUSAMMENSETZUNG
PROCÉDÉ POUR RÉPARER UN ESPACE ANNULAIRE, ET COMPOSITION DE GEL DE COLLAGÈNE

(30) Priority: 03.12.2013 US 201361911325 P
(43) Date of publication of application: 12.10.2016
(73) Proprietor: Cornell University, Ithaca, NY 14850 (US)
(72) Inventor: BORDE, Brandon, Valley Stream, NY 11580 (US); GRUNERT, Peter, Seattle, WA 98101 (US); HARTL, Roger, New York, NY 10065-8805 (US); BONASSAR, Lawrence, Ithaca New York 14850 (US)
(74) Representative: Potter Clarkson
(86) International application number: PCT/US2014/068395
(87) International publication number: WO 2015/084972

(56) References cited:
- WO-A2-2008/013962
- US-A1- 2004 001 251
- US-A1- 2008 004 214
- US-A1- 2009 149 923
- US-A1- 2013 079 881
- US-B2- 7 285 363
- GRUNERT ET AL.: "Annular repair using high-density collagen: An in vivo study", EUROPEAN CELLS AND MATERIALS, vol. 26, no. 8, 6 December 2013 (2013-12-06), pages 15, XP055354735

## Description

This invention was made with government support under grant number 1F31ARO64695-01 awarded by the National Institutes of Health. The government has certain rights in this invention.

### FIELD OF THE INVENTION

The present invention relates to a collagen gel composition for use in repairing an annulus by injecting the composition into, around, or near an annulus repair site under conditions to repair an annulus, the collagen gel composition comprising: collagen gel and a cross-linking agent; wherein the cross-linking agent is riboflavin; wherein the cross-linking agent is included in the composition in an amount sufficient to cause cross-linking of the collagen gel and enable integration of the collagen gel composition with tissue that comes into contact with the collagen gel composition and the collagen is included in the composition at a concentration of 10 to 180 mg/ml.

### BACKGROUND OF THE INVENTION

Low back pain is often a result of a bulging or herniated intervertebral disc ("IVD"). Partial discectomies alleviate the pain of a damaged IVD; however, the resulting annular defect is often left untreated. This increases the likeliness of recurrent disc herniations through the open defect. Annulus repair strategies have been devised to address a range of defects in damaged AF tissue. These therapies aim to mechanically close lesions in the AF to prevent prolapse and perhaps slow or inhibit degeneration.

The types of annulus closure treatments that are in development range from purely mechanical methods to tissue engineering strategies, with varying degrees of success. Many mechanical strategies are already commercially available (i.e., suture, barrier, plug). The multiple purse string suture ("MPSS") method for closing the annulus fibrosus employs a system of sutures and anchors to pull the remaining tissue together. The MPSS is developed into a product called Xclose. There are also implantable barriers that physically bridge the defect, such as the Intrinsic Barricaid and New-Vert OSA system. A research group has also developed barbed plugs that can be inserted into the annulus defect. Although these methods are commercially available, they have numerous drawbacks and limitations. Suture methods introduce small punctures around the initial defect through which the anchors are introduced. Due to the nature of suturing, these smaller punctures are placed under tension. Also, if the initial defect is large, suturing may not be enough to completely close the gap in the annulus. Covering the defect with a barrier requires that the construct be mechanically anchored to surrounding tissue, usually inserted into the adjacent vertebral bone which causes damage to the bone and possibly altered loading on the damaged disc. Repair methods such as the barrier and plugs are fabricated using metal and plastics which always come with a risk of catastrophic failure. The barbed plugs broke into pieces when tested in a sheep model. The resulting shards can cause further damage to the remaining disc as well as the surrounding disc space. None of these mechanical methods provide for lost tissue at the defect side or provide a substrate for tissue growth.

Tissue-engineered fibrin hydrogels are being developed for annulus repair. These gels are able to adhere; however, their potential for biological healing is unknown. Furthermore, the native annulus is comprised of type I collagen, so introduction of fibrin (a factor commonly associated with blood clotting and scar tissue) induced unwanted side effects when tested *in vivo.*

Repair of annular defects could significantly improve treatment of degenerative spinal diseases (Bron et al., "Repair, Regenerative and Supportive Therapies of the Annulus Fibrosus: Achievements and Challenges," Eur. Spine J. 18:301-13 (2009)). Open defects compromise the ability of the AF to contain nuclear tissue in the disc space, thereby increasing the likelihood of reherniation and progressive degeneration after discectomies (Lebow et al., "Asymptomatic Same-Site Recurrent Disc Herniation After Lumbar Discectomy: Results of a Prospective Longitudinal Study with 2-Year Serial Imaging," Spine 36:2147-51 (2011); Ambrossi et al., "Recurrent Lumbar Disc Herniation After Single-Level Lumbar Discectomy: Incidence and Health Care Cost Analysis," Neurosurgery 65:574-8 (2009); McGirt et al., "A Prospective Cohort Study of Close Interval Computed Tomography and Magnetic Resonance Imaging After Primary Lumbar Discectomy: Factors Associated with Recurrent Disc Herniation and Disc Height Loss," Spine 34:2044-51 (2009); Carragee et al., "A Prospective Controlled Study of Limited Versus Subtotal Posterior Discectomy: Short-Term Outcomes in Patients with Herniated Lumbar Intervertebral Discs and Large Posterior Anular Defect," Spine 31:653-7 (2006); Carragee et al., "Clinical Outcomes After Lumbar Discectomy for Sciatica: The Effects of Fragment Type and Anular Competence," J. Bone Joint Surg. Am. 85-A: 102-8 (2003)). Furthermore, there has been concern that annular puncture for therapeutic or diagnostic procedures accelerates the progression of degenerative disc disease and promotes nuclear tissue extrusion (Carragee et al., "2009 ISSLS Prize Winner: Does Discography Cause Accelerated Progression of Degeneration Changes in the Lumbar Disc: A Ten-Year Matched Cohort Study," Spine 34:2338-45 (2009)). Successful treatment of puncture defects could inhibit these degenerative changes.

Annular defects persist because of the very limited intrinsic healing capability of the AF, which does not significantly improve upon simple mechanical closure (Bron et al., "Repair, Regenerative and Supportive Therapies of the Annulus Fibrosus: Achievements and Challenges," Eur. Spine J. 18:301-13 (2009); Melrose et al., "Recent Advances in Annular Pathobiology Provide Insights into Rim-Lesion Mediated Intervertebral Disc Degeneration and Potential New Approaches to Annular Repair Strategies," Eur. Spine J. 17:1131-48 (2008); Bron et al., "Biomechanical and In Vivo Evaluation of Experimental Closure Devices of the Annulus Fibrosus Designed for a Goat Nucleus Replacement Model," Eur. Spine J. 19:1347-55 (2010); Fazzalari et al., "Mechanical and Pathologic Consequences of Induced Concentric Anular Tears in an Ovine Model," Spine 26:2575-81 (2001); Hampton et al., "Healing Potential of the Anulus Fibrosus," Spine 14:398-401 (1989)). As a result, several research groups have investigated using biological materials for annular repair.

Rigid implants have been studied by one group (Vadala et al., "Bioactive Electrospun Scaffold for Annulus Fibrosus Repair and Regeneration," Eur. Spine J. 21(suppl 1):S20-6 (2012)) using tissue-engineered AF constructs *in vitro* and by another group (Ledet et al., "Small Intestinal Submucosa for Anular Defect Closure: Long-Term Response in an In Vivo Sheep Model," Spine 34:1457-63 (2009)) using small intestinal submucosa *in vivo.* Implanted submucosa tissue reduced degenerative changes after annulotomy in sheep spine. Schek et al., "Genipin-Crosslinked Fibrin Hydrogels as a Potential Adhesive to Augment Intervertebral Disc Annulus Repair," Eur. Cell. Mater. 21:373-83 (2011) studied injectable biomaterials with genipin cross-linked fibrin hydrogels. Fibrin integrated with sections of human AF tissue, showing promising biomechanical and cell-seeding properties *in vitro.* Injectable high-density collagen ("HDC") gels were tested and it was found that HDC can partially restore mechanical function to a needle-punctured rat-tail AF *in vitro* (Borde et al., "Repair of Defects in the Rat Tail Annulus Fibrosus Using Injectable High Density Collagen Gels," Paper presented at: Orthopedic Research Society, Annual Meeting; San Francisco, CA (2012)). However, no studies have reported the use of injectable biomaterials to treat annular defects *in vivo.*

Annular defects induced by needle puncture lead to predictable patterns of disc degeneration in the rat-tail spine (Keorochana et al., "The effect of Needle Size Inducing Degeneration in the Rat Caudal Disc: Evaluation Using Radiograph, Magnetic Resonance Imaging, Histology, and Immunohistochemistry," Spine J. 10:1014-23 (2010); Han et al., "A Simple Disc Degeneration Model Induced by Percutaneous Needle Puncture in the Rat Tail," Spine 33:1925-34 (2008)). In such models, degeneration is initiated by extrusion of nucleus pulposus tissue through the puncture defect. Although the needle puncture model has been frequently used to test biological materials for intervertebral disc regeneration (Zou et al., "Efficacy of Intradiscal Hepatocyte Growth Factor Injection for the Treatment of Intervertebral Disc Degeneration," Mol. Med. Rep. 8:118-22 (2013); Zhang et al., "Intradiscal Injection of Simvastatin Retards Progression of Intervertebral Disc Degeneration Induced by Stab Injury," Arthritis Res. Ther. 11:R172 (2009); Gullung et al., "Platelet-Rich Plasma Effects on Degenerative Disc Disease: Analysis of Histology and Imaging in an Animal Model," Evid. Based Spine Care J. 2:13-8 (2011); Sheikh et al., "In Vivo Intervertebral Disc Regeneration Using Stem Cell-Derived Chondroprogenitors," J. Neurosurg. Spine 10:265-72 (2009)), no study yet has used this model to investigate repair of induced defects to prevent disc degeneration.

The present invention is directed to overcoming these and other deficiencies in the art.
US2004/010251 describes a therapeutic method for treating mammalian intervertebral discs. A preparation of cross-linked collagen is injected under pressure into the intradiscal space. The intervertebral distance in injected discs is immediately increased by the treatment. At least some mechanical properties of the treated vertebral column are preserved or partially restored. The method may be used to relieve back pain in patients, to increase patient height and to stabilized the spinal column. The therapeutic method may result in at least a partial regeneration of the nucleus pulposus, and/or development of cartilaginous or fibrocartilaginous tissues or dense fibrous tissues.
WO2008/13962 describes a method and a composition for photochemical cross linking of collagen by photoactive agent in-vivo are presented. The method includes a non-toxic photoactive formulation of the composition with collagen, which is administered to treatment area locally; followed by irradiation with suitable wavelength. In one of the embodiment liposomal formulated mTHPC is added to the collagen and is irradiated with a 652 nm laser, resulting in producing efficient collagen scaffolds with strengthen and stabilized microstructure, thus improving the physiochemical properties of the collagen scaffold.

### SUMMARY OF THE INVENTION

The present invention relates to a collagen gel composition for use in repairing an annulus that includes collagen gel and a cross-linking agent; wherein the cross-linking agent is riboflavin; wherein the cross-linking agent is included in the composition in an amount sufficient to cause cross-linking of the collagen gel and enable integration of the collagen gel composition with tissue that comes into contact with the collagen gel composition and the collagen is included in the composition at a concentration of 10 to 180 mg/ml. The collagen gel composition is injected into, around, or near an annulus repair site under conditions to repair the annulus. The cross-linking agent is included with or added to the collagen gel in an amount sufficient to cause cross-linking of the collagen gel and enable integration of the collagen gel composition with tissue near where said collagen gel composition is injected.

The cross-linking agent is included in the composition in an amount sufficient to cause cross-linking of the collagen gel and enable integration of the collagen gel composition with tissue that comes into contact with the collagen gel composition.

The gel composition is injectable and of low viscosity upon delivery, and thus can be delivered to a variety of shapes and sizes to partially or completely seal an annulus defect. Delivery of the gel composition does not cause damage to any of the surrounding tissue and has been shown to integrate with neighboring native disc tissue. Furthermore, since the method involves injecting a gel composition of a biological material, there is no chance of catastrophic failure leaving destructive shards in the surrounding disc space. As mentioned *supra*, the native annulus is comprised of mostly type-I collagen, therefore delivery of cross-linked type-I collagen gel to repair the region provides a substrate for long-term tissue growth and biological repair of the defect.

In the examples set forth *infra*, high-density type I collagen is shown to repair annular defects for up to 5 weeks after induction of a needle-puncture defect in the AF of the rat-tail spine. This capability correlates positively with RF cross-linking. The needle-punctured rat-tail has been shown to be a suitable *in vivo* model for studying biomaterials for annular repair. The goal of the experiments set forth in the Examples *infra* was to evaluate the ability of HDC to repair a needle-puncture AF defect in the rat-tail spine. Specifically, the goal was to test whether injected HDC gel can prevent nuclear tissue extrusion and/or consequent IVD degeneration, as determined by histological and radiological outcomes. In addition, it was assessed whether cross-linking of injected collagen influences the repair process. For that purpose, riboflavin ("RF"), a photoactive initiator of collagen cross-linking, was added in different concentrations. The rat-tail model was used to screen these various compositions of collagen gels.

### BRIEF DESCRIPTION OF THE DRAWINGS

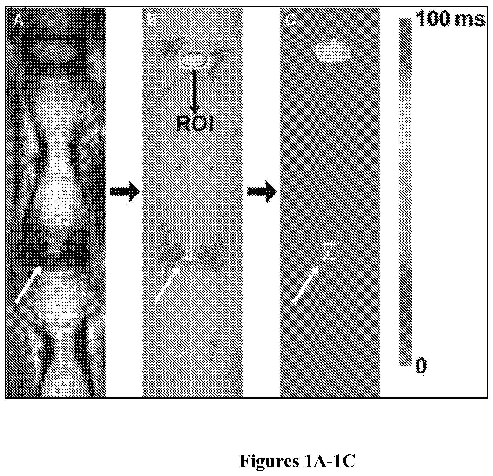

Figures 1A-C are photographs of a method used to determine nucleus pulposus size according to T2-RT. Figure 1A shows a T2-weighted magnetic resonance image of a rat-tail with a needle-punctured disc (white arrow). Figure 1B shows a matching image with T2-RT measurements displayed as a color map. An ROI was drawn within the NP of the proximal healthy adjacent disc. A subtraction threshold was set at 2 standard deviations below the T2-RT mean of the ROI. The image of Figure 1C shows that all voxels below the threshold were subtracted. Subsequently, the remaining voxels in the disc space were counted. ROI indicates region of interest.

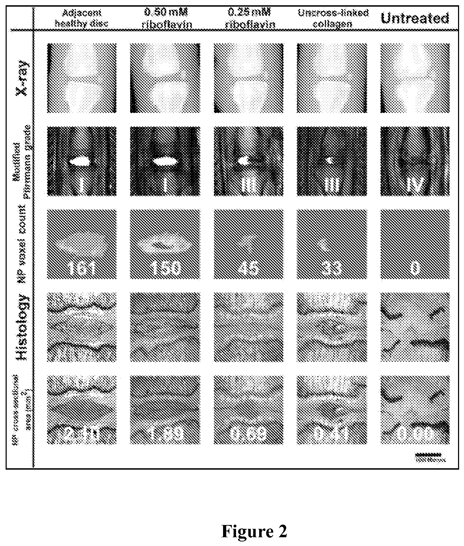

Figure 2 is a chart of images showing examples of five-week outcomes of all punctured groups. The displayed specimen from the 0.5 mM RF group shows only a slight reduction in NP size when compared with healthy discs, according to the NP voxel count and histological cross-section measurements. The IVD from the 0.25 mM RF group shows a reduced nuclear size. The NP appears still homogeneous and hyperintense but has lost its oval shape. The decreased disc height, which is also seen on radiograph, results in a Pfirrmann grade of III. The uncross-linked collagen-injected IVD shows a greater reduction in NP size. The NP is still hyperintense but appears more heterogeneous. The untreated disc shows terminal degenerative changes: a black disc on magnetic resonance images combined with collapsed disc space. The NP tissue has been completely replaced with connective tissue. Endplate damage is visible. NP indicates nucleus pulposus.

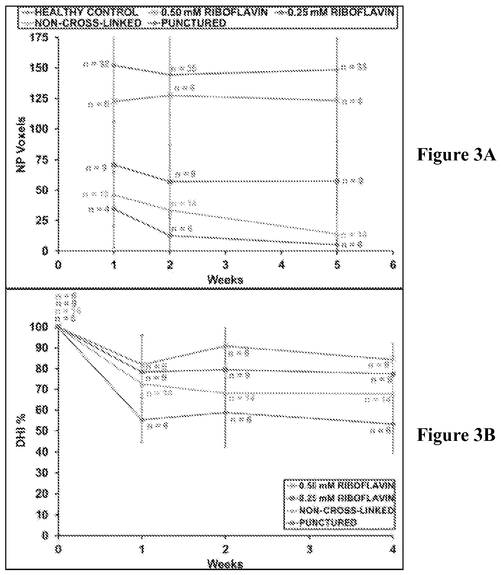

Figures 3A-B are graphs showing nuclear size and disc height measurements. The graph of Figure 3A shows nuclear size according to NP voxel count measurements. The 0.5 mM RF group showed slightly fewer NP voxels than did healthy discs over 5 weeks. Voxel counts of both cross-linked groups remained constant between the second and fifth weeks. Animals injected with uncross-linked collagen gel showed a continuous drop in NP voxels over 5 weeks. The untreated group showed the lowest voxel count numbers at all time points. Figure 3B shows disc height measurements correlated with NP voxel count results. The 0.5 mM RF group retained 88% of its initial disc height; the untreated group dropped to 55%. Variations in within groups resulted from magnetic resonance imaging scheduling constraints. NP indicates nucleus pulposus; DHI, disc height index.

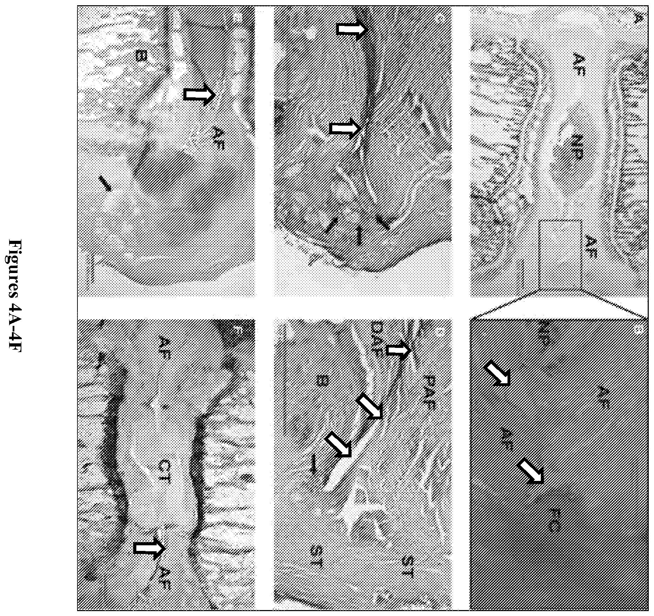

Figures 4A-F are photographs of different histological outcomes of punctured IVDs after 5 weeks. Figure 4A shows Safranin O stain, 0.25 mM RF group. The disc displays an ovular NP of standard size. The needle-puncture defect and fibrous cap are outlined by the box. Figure 4B shows higher magnification under polarized light. The needle-puncture defect is clearly visible (white arrows). The inner two-thirds of the AF lamellae remain separated; there is no sign of tissue repair. The outer third of the defect is bridged by a fibrous cap ("FC"). The nonbirefringent FC matrix seems to infiltrate the bright, birefringent AF fibers. Figure 4C shows Alcian blue stain of the 0.25 mM RF group. Only a thin fibrous string (black arrows) bridges the annular defect (white arrows). Figure 4D shows Alcian blue stain of the uncross-linked group. No fibrous tissue is visibly repairing the defect (white arrows). Distal annular fibers ("DAF") and proximal annular fibers ("PAF") remain separated and infiltrate the surrounding scar tissue ("ST"). The black arrow points to sequestered NP material. Figure 4E shows Alcian blue stain of the uncross-linked group. NP tissue (black arrow) extrudes through the annular defect into the paravertebral space. The AF shows multiple fissures (white arrow). Figure 4F shows untreated control IVD. The needle-puncture defect (arrow) induced severe degenerative changes. There is no organized AF tissue visible; the NP has been replaced with connective tissue. Key: B, endplate bone; CT, computer tomography.

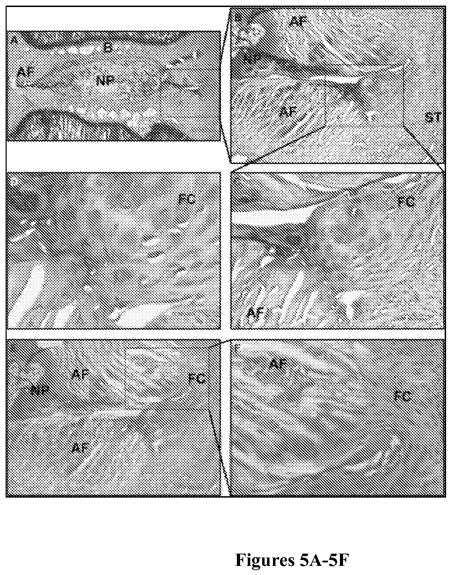

Figures 5A-F are photographs of Safranin O stain of punctured IVD, 0.5 mM RF group after 5 weeks. Figure 5A shows low magnification. The NP displays standard size and ovular shape. A clear border separates the NP from the AF and endplate bone B. The box marks the needle puncture defect. Figure 5B is higher magnification showing the needle puncture defect piercing through every layer of the AF. A fibrous cap, marked by the box, bridges the annular defect at the outer portion of the AF. The matrix of this fibrous tissue stains more intensely with Safranin O than does the surrounding ST. Figures 5C and 5D show higher magnification of the fibrous cap, which appears to be infiltrating AF fibers. Figures 5E and 5F show the same specimen as in A to C, viewed under polarized light. The bright birefringent AF fibers mesh with the nonbirefringent tissue of the fibrous cap. Key: AF indicates annulus fibrosus; NP, nucleus pulposus; ST, scar tissue; FC, fibrous cap.

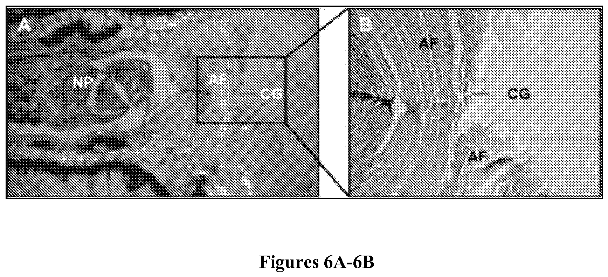

Figures 6A-B are photographs showing the fate of the collagen gel. Figure 6A shows Safranin O stain, polarized light immediately after collagen gel ("CG") injection. High-density collagen (HDC) distributes in the paravertebral space, covering the AF defect. In contrast to the AF, HDC is nonbirefringent under polarized light, indicating a lower tissue organization. Figure 6B shows higher magnification. The puncture defect is visible (arrows). CG appears as an amorphous homogenous tissue that stains slightly positive for Safranin O. It does not migrate into the AF defect. Key: NP indicates nucleus pulposus; AF, annulus fibrosus; CG, collagen gel.

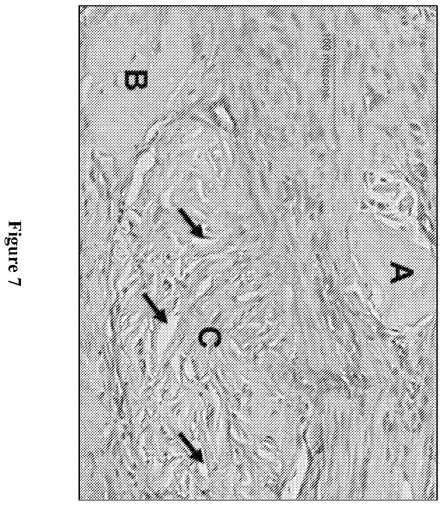

Figure 7 is a photograph showing the fate of the collagen using Alcian blue stain in the paravertebral space 5 weeks after collagen injection. Three islets of amorphous homogenous collagen are surrounded by scar tissue. Islet A shows no cell infiltration, islet B very limited. Islet C shows a high degree of fibroblast infiltration. The cells appear to reorganize the tissue, as indicated by resorptive zones (black arrows) within the collagen tissue. There is no sign of an inflammatory response.

### DETAILED DESCRIPTION OF THE INVENTION

The present invention relates to a collagen gel composition for use in repairing an annulus that includes collagen gel and a cross-linking agent; wherein the cross-linking agent is riboflavin; and wherein the cross-linking agent is included in the composition in an amount sufficient to cause cross-linking of the collagen gel and enable integration of the collagen gel composition with tissue that comes into contact with the collagen gel composition and the collagen is included in the composition at a concentration of 10 to 180 mg/ml. The collagen gel composition is injected into, around, or near an annulus repair site under conditions to repair the annulus. The cross-linking agent is included with or added to the collagen gel in an amount sufficient to cause cross-linking of the collagen gel and enable integration of the collagen gel composition with tissue near where said collagen gel composition is injected.

The cross-linking agent is included in the composition in an amount sufficient to cause cross-linking of the collagen gel and enable integration of the collagen gel composition with tissue that comes into contact with the collagen gel composition.

Intervertebral discs separate the spinal vertebrae from one another and act as natural shock absorbers by cushioning impacts and absorbing the stress and strain transmitted to the spinal column. Intervertebral disc tissues are primarily composed of three regions, the end plates, the annulus fibrosus ("AF") and the nucleus pulposus ("NP"). The annulus fibrosus is a tough collagen-fiber composite that has an outer rim of type I collagen fibers surrounding a less dense fibrocartilage and a transitional zone. These collagen fibers are organized as cylindrical layers. In each layer the fibers are parallel to one another; however, the fiber orientation between layers varies between 30 and 60 degrees. This organization provides support during torsional, bending, and compressive stresses on the spine. The end plates, which are found at the upper and lower surfaces of the disc, work in conjunction with the annulus fibrosus to contain the gel-like matrix of the nucleus pulposus within the intervertebral disc. The nucleus pulposus is made up of a soft matrix of proteoglycans and randomly oriented type II collagen fibers in water. The proteoglycan and water content are greatest at the center of the disc and decrease toward the disc periphery.

The collagen gel composition is used to repair, *e.g.*, the annulus fibrosus structure of an IVD. As used herein, the term "repair" refers to any correction, reinforcement, reconditioning, remedy, making up for, making sound, renewal, mending, patching, or the like that restores, preserves, and/or augments function and/or structure. Accordingly, the term "repair" can also mean to correct, to reinforce, to recondition, to remedy, to make up for, to make sound, to renew, to mend, to patch, to preserve (*i.e.*, from further injury or degradation), or to otherwise restore function and/or structure. "Repair" includes full repair and partial repair of an intervertebral disc annulus defect. Furthermore, the term "repair" also includes treatment, prevention, or amelioration of at least one symptom associated with or caused by an intervertebral disc annulus defect. By "treatment," "prevention," or "amelioration" is meant delaying or preventing the onset, reversing, alleviating, ameliorating, inhibiting, slowing down or stopping the progression, aggravation or deterioration the progression or severity of a condition associated with an intervertebral disc annulus defect or injury.

The collagen gel composition is to "repair" the AF of an intervertebral disc, and following treatment to repair the annulus, at least about 75%, 76%, 77%, 78%, 79%, 80%, 81%, 82%, 83%, 84%, 85%, 86%, 87%, 88%, 89%, 90%, or more of the disc height is maintained after a period of days, weeks, months, or years.

The collagen gel composition (described *infra*) is injected into, around, or near an annulus repair site under conditions effective to repair the annulus. The injection of the composition can be performed, for example, using a guide needle (but a guide needle is not required) inserted into the annulus fibrosus of the affected intervertebral disc. An injection needle can then be inserted through the guide needle. It may or may not be necessary to penetrate the annulus fibrosus with the injection needle. The location of the tip of the guide needle or the location of the tip of the injection needle can be verified using imaging technology, *e.g.*, fluoroscopy, magnetic resonance imaging, computed tomography, or any other similar technology well known in the art.

In one embodiment, injecting does not cause damage to surrounding tissue.

In another embodiment, injecting results in the injected gel composition integrating with native tissue of the annulus.

The collagen gel composition may be carried out in any mammal, including humans.

Several types of collagen exist. Types I, II, III, and IV of collagen have been mainly used as sources of biomaterials. Type I collagen can be found in most connective tissues, is the most abundant collagen type in a living organism, and is found in tendons, corium, and bone. Industrially, in many cases, collagen can be extracted from those portions of an organism. Type II is a collagen that forms cartilage. Type III collagen is found in small quantities in the same tissues as type I collagen. Type IV collagen forms a basal membrane. Types I, II, and III are present as collagen fibers in living organisms, and have a main function of maintaining the strength of a tissue or organ. Type IV, which does not have ability for fibrogenesis, forms a net-like assembly constructed of four molecules and may take part in cell differentiation in a basal membrane. As used herein, the term "collagen" refers to any one or more of the types of collagen known in the art or to be discovered.

The collagen in the collagen gel composition may be type I collagen. This is the same type of collagen as the natural AF. When type I collagen is delivered to a tissue that already contains type I collagen, the chances of immune response to the gel composition (following injection) and any negative interaction with the surrounding tissue is eliminated.

The collagen may be soluble or insoluble and may be derived from any tissue in any animal. For example, collagen gel may be prepared using collagen derived from skin and tendon, including rat tail tendon, calf skin collagen, and/or calf extensor tendon.

Collagen gel is included in the composition of the present invention at a concentration of about 10-180 mg/ml, about 20-160 mg/ml, about 30-140 mg/ml, about 40-120 mg/ml, about 50-100 mg/ml, about 60-80 mg/ml, greater than about 50 mg/ml, greater than about 100 mg/ml, greater than about 150 mg/ml, less than about 200 mg/ml, less than about 150 mg/ml, less than about 100 mg/ml, or less than about 50 mg/ml.

In the composition of the present invention, a cross-linking agent is included, which agent is included in an amount sufficient to cause cross-linking of the collagen gel. The agent may also cause some cross-linking with surrounding collagen fibers. This means, for example, that there is good adhesion of injected gel with tissue surrounding the defect. In other words, the composition of the present invention, when injected, stably integrates into surrounding tissue. According to one embodiment, this means that the composition of the present invention is, upon injection into an annulus repair site, able to stay in place at the repair site, because it integrates with surrounding tissue.

The cross-linking of collagen-based materials may be used to suppress the antigenicity of the material in order to prevent the hyperacute rejection reaction.

Cross-linking may also be used to improve mechanical properties and enhance resistance to degradation.

Various cross-linking methodologies may be used to cross-link collagen gel compositions. The cross-linking may be photo-induced, using UV or visible light treatment. Various aspects of the light exposure can be varied such as time of exposure (0-600 or more seconds) and intensity (up to 2,000 mw/cm²) of the light. UV irradiation (at, *e.g.*, 200-300 nm) is a rapid and easily controlled means of increasing the mechanical strength of collagen fibres and does not produce any toxic by-products which require subsequent removal by washing the biomaterial. The collagen gel may comprise hydroxyl containing components such as polysaccharides or GAGs, which may optimize UV and/or visible cross-linking treatment. The collagens may be cross-linked using visible light in the presence of photosensitizer dyes such as methylene blue and rose bengal stain.

Cross-linking may also be carried out by radiation treatment, using electron beam and/or gamma rays. Radiation-induced cross-linking does not require the use of cytotoxic cross-linkers and no subsequent treatment steps are necessary. Another advantage of radiation-induced cross-linking is that gamma rays can penetrate the biomaterial to depths that may not be achieved using other methodologies such as visible light or UV light-induced cross-linking.

In the collagen gel compositions of the invention the cross-linking agent is riboflavin. A cross-linking agent (*e.g.*, riboflavin) mixed into the gel helps promote cross-linking not only within the collagen gel, but also with the surrounding native collagen fibers. The collagen gel composition of the present invention polymerizes in the region of the defect, which allows collagen in the composition to exploit natural interactions with the surrounding native collagen to promote cohesion and mechanical healing at a defect site. Polymerization in a defect site also allows the gel to spread and conform to different defect shapes and sizes.

The cross-linking agent may be included in the composition at a concentration of up to about 1.5 mM, about 2.0 mM, about 2.5 mM, about 3.0 mM, about 3.5 mM, about 4.0 mM, about 4.5 mM, about 5.0 mM, about 5.5 mM, about 6.0 mM, about 6.5 mM, about 7.0 mM, or about 7.5 mM, or at a concentration greater than about 7.5 mM.

The collagen gel composition of the present invention is highly mechanically, chemically, and biologically tunable. The collagen is processed in a way that allows varying collagen density and cross-linker concentration, both of which affect mechanical properties. One can also add cells during, before, and/or after the mixing process, increasing the versatility of the composition. Various growth factors and agents, including therapeutic agents can be added to the composition to elicit specific responses from cells and the surrounding tissue.

The collagen gel composition of the present invention may further comprise living cells. The cells may be stem or progenitor cells, differentiated cells, terminally differentiated cells, or combinations thereof. The cells may be pluripotent or unipotent stem cells, or induced pluripotent stem cells. The cells may be human embryonic stem cells, derived via a technology which does not necessitate the destruction of the human embryo, for example via an established cell line. Mesenchymal stem cells (also referred to as marrow stromal cells, multipotent stromal cells, or MSCs) are pluripotent stem cells which can differentiate into a variety of cell types including osteoblasts, tenocytes, chondrocytes, myocytes, adipocytes. These cell types have the ability to generate bone, tendon, ligament, cartilage, muscle, and fat. The cells may be MSCs or any cell within the MSC lineage. Progenitor cells can go through several rounds of cell division before terminally differentiating into a mature cell, and the cells may be these intermediary cells. The cells may be selected from MSCs (marrow stromal cells, mesenchymal stem cells, multipotent stromal cells), chondrocytes, fibrochondrocytes, osteocytes, osteoblasts, osteoclasts, synoviocytes, adipocytes, bone marrow cells, mesenchymal cells, stromal cells, genetically transformed cells, or combinations thereof. The cells may be autologous or heterologous.

The cells may include one or more of the following: embryonic stem cells; precursor cells derived from adipose tissue and/or bone marrow; peripheral blood progenitor cells; stem cells and/or non-stem cells isolated from adult tissue and/or juvenile; genetically transformed cells; a combination of chondrocytes and other cells; a combination of osteocytes and other cells; a combination of synoviocytes and other cells; a combination of bone marrow cells and other cells; a combination of mesenchymal cells and other cells; a combination of stromal cells and other cells; a combination of stem cells and other cells; a combination of embryonic stem cells and other cells; a combination of precursor cells isolated from adult tissue and other cells; a combination of peripheral blood progenitor cells and other cells; a combination of stem cells isolated from adult tissue and other cells; and a combination of genetically transformed cells and other cells.

Although human cells are preferred for use in the composition and method of the present invention, the cells are not limited to cells from human sources. Cells from other mammalian species including, but not limited to, equine, canine, porcine, bovine, feline, caprine, and ovine sources may be used. Murine cells, cells from rodent sources, may also be used. Cell donors may vary in development and age. Cells may be derived from donor tissues of embryos, neonates, or older individuals including adults.

The living cells may be selected from chondrocytes, fibroblasts, stem cells, and combinations thereof.

Cells included in the collagen gel composition of the present invention may be engineered, *e.g.*, to express a particular protein. For example, nucleic acid molecules encoding a certain protein can be incorporated into host cells using conventional recombinant DNA technology. Generally, this involves inserting the DNA molecule into an expression system to which the DNA molecule is heterologous (*i.e.*, not normally present). The heterologous DNA molecule is inserted into the expression system or vector in sense orientation and correct reading frame. The vector contains the necessary elements (promoters, suppressers, operators, transcription termination sequences, etc.) for the transcription and translation of the inserted protein-coding sequences. A recombinant gene or DNA construct can be prepared prior to its insertion into an expression vector. For example, using conventional recombinant DNA techniques, a promoter-effective DNA molecule can be operably coupled 5' of a DNA molecule encoding the protein and a transcription termination (*i.e.*, polyadenylation sequence) can be operably coupled 3' thereof.

Polynucleotides encoding a desired protein can be inserted into an expression system or vector to which the molecule is heterologous. The heterologous nucleic acid molecule is inserted into the expression system or vector in proper sense (5'→3') orientation relative to the promoter and any other 5' regulatory molecules, and correct reading frame. The preparation of the nucleic acid constructs can be carried out using standard cloning methods well known in the art as described by Sambrook & Russell, Molecular Cloning: A Laboratory Manual (Cold Springs Laboratory Press, 2001). U.S. Patent No. 4,237,224 to Cohen and Boyer, also describes the production of expression systems in the form of recombinant plasmids using restriction enzyme cleavage and ligation with DNA ligase.

Suitable expression vectors include those which contain replicon and control sequences that are derived from species compatible with the host cell. For example, if *E. coli* is used as a host cell, plasmids such as pUC19, pUC18, or pBR322 may be used. When using insect host cells, appropriate transfer vectors compatible with insect host cells include, pVL1392, pVL1393, pAcGP67, and pAcSecG2T, which incorporate a secretory signal fused to the desired protein, and pAcGHLT and pAcHLT, which contain GST and 6xHis tags (BD Biosciences, Franklin Lakes, NJ). Suitable viral vectors include adenoviral vectors, adeno-associated viral vectors, vaccinia viral vectors, nodaviral vectors, and retroviral vectors. Other suitable expression vectors are described in Sambrook and Russell, Molecular Cloning: A Laboratory Manual (Cold Springs Laboratory Press, 2001). Many known techniques and protocols for manipulation of nucleic acids, for example in preparation of nucleic acid constructs, mutagenesis, sequencing, introduction of DNA into cells and gene expression, and analysis of proteins, are described in detail in Current Protocols in Molecular Biology (Fred M. Ausubel et al. eds., 2003).

Different genetic signals and processing events control many levels of gene expression (*e.g.*, DNA transcription and messenger RNA ("mRNA") translation) and subsequently the amount of desired protein that is produced and expressed by the host cell. Transcription of DNA is dependent upon the presence of a promoter, which is a DNA sequence that directs the binding of RNA polymerase, and thereby promotes mRNA synthesis. Promoters vary in their "strength" (*i.e.*, their ability to promote transcription). For the purposes of expressing a cloned gene, it is desirable to use strong promoters to obtain a high level of transcription and, hence, expression. Depending upon the host system utilized, any one of a number of suitable promoters may be used. Common promoters suitable for directing expression in mammalian cells include, without limitation, SV40, MMTV, metallothionein-1, adenovirus Ela, CMV, immediate early, immunoglobulin heavy chain promoter and enhancer, and RSV-LTR. The promoters can be constitutive or, alternatively, tissue-specific or inducible.

The composition of the present invention is injectable. Since the collagen gel composition of the present invention is injectable, it can be delivered to different defect sites with minimally invasive surgical techniques and standard medical equipment. Other AF repair compositions and techniques require larger surgery sites to allow for anchoring to surrounding tissue. Also, unlike the composition of the present invention, other existing AF repair techniques cannot conform to different defect shapes and sizes prompting surgeons to remove more natural tissue to make a uniform defect.

The collagen gel composition of the present invention has low viscosity upon delivery and polymerizes in any defect independent of defect size and shape. Furthermore, the use of, *e.g.*, riboflavin to cross-link *in situ* is new and unique in that a more mechanically stable gel can form without compromising the gel/AF interface.

### EXAMPLES

The examples below are intended to exemplify the practice of the present invention, the invention is limited to the scope of the appended claims.

### Materials and Methods

### Study Groups

42 male, 10- to 12-week-old, inbred, nude, athymic rats were used for this study. Animals were divided into 4 groups. The first group of 14 was needle-punctured and injected with HDC of 15 mg/mL concentration. The second group of 15 was punctured and injected with RF cross-linked HDC (15 mg/mL): 9 with 0.25 mM RF, and 6 with 0.5 mM RF. A third group of 6 was punctured and left untreated to serve as control. All the animals in these 3 groups were killed after 5 weeks. Tails were collected and used for histological analysis. A fourth group of 6 animals was killed; tails were collected and subsequently needle-punctured and injected with HDC. This group was used to study the morphology and distribution of the collagen directly after injection.

All animals were killed with carbon dioxide pursuant to standard protocols from the American Veterinary Medical Association.

The study was approved by and undertaken in accordance with guidelines outlined by the Hospital of Special Surgery Institutional Animal Care and Use Committee and New York State.

### Collagen Gel Preparation

Collagen was harvested and reconstituted from rat-tail tendon as previously described (Cross et al., "Dense Type I Collagen Matrices That Support Cellular Remodeling and Microfabrication for Studies of Tumor Angiogenesis and Vasculogenesis In Vitro," Biomaterials 31:8596-607 (2010); Bowles et al., "Self-Assembly of Aligned Tissue-Engineered Annulus Fibrosus and Intervertebral Disc Composite Via Collagen Gel Contraction," Tissue Eng Part A 16:1339-48 (2010). Fibers were digested in 0.1% acetic acid, frozen for 48 hours, lyophilized, and reconstituted at 20 mg/mL in 0.1% acetic acid. Immediately before delivery, acidic collagen solutions were mixed with working solutions containing 10x Dulbecco's Phosphate Buffered Saline ("DPBS"), 1N NaOH, and 1x DPBS to initiate polymerization of final collagen gels at 15 mg/mL. For RF groups, RF was added to 1x DPBS at the desired concentration. Gels with RF were exposed to blue light with a 480-nm wavelength for 40 seconds after injection to initiate cross-linking.

### Open Needle Puncture and Collagen Injection

The target level between the 3rd and 4th vertebra of the tail was localized under radiographical control. The animals were anesthetized and placed in the prone position. A 2-cm longitudinal skin incision was made over the marked level. The AF was then exposed, and great care was taken to preserve it without damage. Subsequently, the AF was punctured using an 18-gauge needle attached to a syringe containing the collagen gel. Approximately 0.5 mL of the gel was injected around the defect immediately after puncture. The gel was cured *in situ* when RF was added to the collagen. The control group was punctured and left untreated. To standardize the puncture technique, the needle was always inserted in the same orientation (with the needle bevel up) and to the same depth (until the bevel completely penetrated the AF).

### Quantitative Magnetic Resonance Imaging

Quantitative imaging was utilized to assess the size of the NP according to the number of magnetic resonance imaging ("MRI") voxels that composed it, here labeled NP voxel count. Magnetic resonance image was obtained on a 7T MRI (Bruker 7T USR Preclinical MRI System) at 1, 2, and 5 weeks postpuncture (Figure 1A).

A sagittal multislice multiecho pulse sequence (TR = 2000 ms, TE = 12 ms, NEX = 2, number of echoes = 12, echo spacing = 12 ms, slice thickness = 1 mm, and matrix size = 320 × 320, resolution: 125 µm × 125 µm × 1 mm) was used to create a T2 map on the basis of fitting semilog plots of T2-signal intensity versus relaxation time for the 12 acquired echoes. Bruker's (Bruker BioSpin Corporation, Billerica, MA) proprietary program TopSpin was used for the fitting process. A color map was assigned to the resulting T2 map (Figure 1B). Next, a standard region of interest ("ROI") measuring approximately 1 mm² was drawn within the center of the NP of the healthy disc proximal to the punctured IVD. The average T2-relaxation time (T2-RT) of that ROI was measured, and this value minus 2 standard deviations was used to set a subtraction threshold for all voxels in that slice (Figure 1B). Voxels with T2 values lower than the threshold were subsequently subtracted (Figure 1C). As a result, only voxels with T2 values representing NP tissue remained in the disc space and were then counted. At each time point, the mean voxel count of punctured discs was compared with the mean voxel count of proximal adjacent healthy IVDs.

### Qualitative MRI Analysis

A sagittal TurboRare sequence (TR = 2017 ms, TE = 60 ms, NEX = 6, echo train length = 12, slice thickness = 1 mm and matrix size = 320 × 320, and resolution: 125 µm × 125 µm × 1 mm) was utilized for qualitative assessments. A modified Pfirrmann scale (Yu et al., "MRI Assessment of Lumbar Intervertebral Disc Degeneration with Lumbar Degenerative Disease Using the Pfirrmann Grading Systems," PLoS One 7:e48074 (2012) was used that outlines 4 grades of degeneration as defined by NP signal intensity, homogeneity, and loss of disc height (Table 1).

**Table 1: Modified Magnetic Resonance Imaging Pfirrmann Grading**

| **Grade** | **Structural Changes Within NP** | **Signal Intensity** | **Intervertebral Disc Height** |
|---|---|---|---|
| I | Homogenous and bright | Hyperintense | Normal |
| II | Heterogeneous | Intermediate | Normal |
| III | Heterogeneous and gray | Intermediate | Decreased |
| IV | Heterogeneous and black | Hypointense | Decreased or collapsed |
| *NP indicates nucleus pulposus.* | | | |

### Disc Height Measurements

Disc heights were obtained in a digital radiographical cabinet. Great care was taken to achieve true lateral radiographs of the index segment. The IVD height was expressed as a disc height index, calculated by dividing disc height by adjacent vertebral body height on the basis of the modified method of Lu et al., "Effects of Chondroitinase ABC and Chymopapain on Spinal Motion Segment Biomechanics. An In Vivo Biomechanical, Radiologic, and Histologic Canine Study," Spine 22:1828-34 (1997). The disc height was measured preoperatively and postoperatively at 1-, 2-, and 5-week time points.

### Histology

After appropriate fixation, tails were decalcified, cut midsagittally, and transferred to 75% ethanol. Segments were embedded in paraffin, then cut to 5 µm thickness, and stained with Alcian Blue and Safranin-O. The size of the NP was measured by drawing an ROI around the nucleus and the cross-sectional area of that ROI was subsequently measured using the Bioquant image program (Figure 2). The average cross-sectional area measurements of punctured discs were compared with those of healthy proximal adjacent IVDs. The Han grading system was used to describe the degenerative stage of the punctured IVDs. This system is based on AF/NP cellularity, morphology, and border (Han et al., "A Simple Disc Degeneration Model Induced by Percutaneous Needle Puncture in the Rat Tail," Spine 33:1925-34 (2008). The grading system ranges from 5 (a healthy disc) to 15 (a terminally degenerated disc).

### Statistics

The NP voxel count and disc height data were evaluated using nested multilevel analysis of variance modeling, with nesting in punctured and healthy adjacent levels, untreated puncture versus collagen injection, and RF concentrations. Analysis was done in JMP 9.0 (SAS Institute Inc., SAS Campus Drive Cary, NC). p≤0.05 indicated a statistically significant correlation between the variables and measured outcomes.

### Example 1 - Qualitative MRI

Five of 6 animals in the 0.5-mM RF group showed no obvious signs of degenerative changes on the 5-week MR images (Figure 2). The modified Pfirrmann grade of that group ranged from I to III (average: 1.3). At the same time point, all animals in the 0.25 mM RF group displayed decreased NP size, yet the nucleus remained homogenous and hyperintense (Figure 2). The Pfirrmann grade ranged from II to III (average: 2.6). The uncross-linked collagen group showed a more heterogeneous, smaller NP with a significantly increased AF/NP ratio (Figure 2). The AF bulged into the nucleus giving it an hourglass shape. All animals had a Pfirrmann grade of III. Five of 6 specimens in the untreated group showed black IVDs, indicating terminal disc degeneration (Figure 2). The Pfirrmann grade ranged from III to IV (average: 3.8).

### Example 2 - NP Voxel Count

The control IVDs had an average NP voxel count ranging from 140 to 153 voxels at 1, 2, and 5 weeks; the voxel count for the 0.5 mM RF group was between 129 (SD +/- 16.3) and 140 (SD +/- 22.5). The 0.25 mM RF group exhibited lower values: 71 voxels (SD +/- 59.14) at week 1 and 57 voxels (SD +/- 56.40) at the second and fifth weeks. The NP voxel counts of both RF groups remained constant between weeks 2 and 5.

The uncross-linked collagen group showed 46 (SD +/- 25) voxels at week 1, which continuously dropped to 14 (SD +/- 15.6) at week 5. The untreated group showed the lowest voxel counts values at each time point, decreasing to 5 (SD +/- 4.17) after 5 weeks (Figure 3A). Overall, HDC gel injection significantly increased voxel count values (P < 0.0001) as compared with untreated discs. Increasing RF concentration (0 mM, 0.25 mM, and 0.5 mM) proved to increase NP voxel count values significantly (P < 0.0001).

### Example 3 - Disc Height Measurements

All punctured IVDs dropped in disc height after needle puncture but remained constant between weeks 2 and 5 (Figure 3B). The 0.5-mM RF group maintained an average of 84% (SD +/- 8.77) of its initial disc height, the 0.25 mM RF group maintained an average of 77% (SD +/- 8.72).

The uncross-linked collagen group maintained 67% (SD +/- 16.86) and the untreated group maintained 53% (SD +/- 13.82) of disc height. Overall, HDC treatment gel significantly improved the disc height index (P = 0.0264).

### Example 4 - The Annular Defect

The needle puncture defect pierced through all AF lamella layers (Figures 4A, 4B and 5A-5C) and was still visible after 5 weeks in all animals. In the control and uncross-linked groups, the AF fibers remained separated and infiltrated the surrounding scar tissue (Figure 4D). No granulation tissue, increased cellularity, or vascularity was visible to indicate an annular repair process. The 0.5-mM RF group showed formation of a fibrous cap at the outer third of the AF that infiltrated annular fibers, bridging both disrupted ends (Figures 5A-5F). This fibrous cap was composed of fibroblasts embedded in a dense collagen matrix that stained more intensely with Safranin-O and Alcian blue than surrounding AF and scar tissue. The 0.25-mM RF group showed formation of either a similar cap (Figures 4A and 4B) or a thin fibrous string repairing the outer part of the AF (Figure 4C). In both cross-linked groups, the inner two-thirds of the AF showed no signs of tissue repair.

### Example 5 - Disc Degeneration

There were no obvious signs of degeneration in the 0.5-mM RF group. IVDs showed an average Han degeneration grade of 6.8. IVDs in the 0.25-mM RF group showed a decline in NP cells with clusters formation and reached an average Han grade of 9.3.

All IVDs in the uncross-linked collage group showed signs of progressed degenerative changes with only residual NP tissue visible in the disc space (Figure 2). The puncture defect branched into the AF, creating multiple fissures. NP tissue herniated through the open defect (Figure 4E). Discs showed a Han grade of 12.8.

All animals in the untreated group showed severe signs of degeneration; the NP was completely replaced with connective tissue. The AF appeared ruptured and disorganized (Figure 4F). Several specimens showed significant endplate damage (Figure 2). The average Han grade was 14.3.

### Example 6 - Histological Cross-Sectional Area

After 5 weeks, healthy NP proximal to punctured IVDs had an average NP cross-sectional area of 1.59 mm². Means of 0.5-mM RF, 0.25-mM RF, and uncross-linked collagen groups were 1.29 mm², 0.78 mm², and 0.78 mm², respectively. No measurable NP tissue remained in the disc space of untreated IVDs.

### Example 7 - Fate of the Injected Collagen

Immediately after injection, the collagen distributed into the paravertebral space covering the outer part of the annular defect but did not migrate into the defect (Figures 6A-6B). HDC appeared as homogenous amorphous tissue that stained weakly with Alcian Blue or Safranin-O. After 5 weeks, residual collagen still appeared homogenous and amorphous and was infiltrated with host fibroblasts to varying degrees. Resorptive zones were visible, indicating reorganization of the injected material by the host cells (Figure 7). No sign of inflammatory or foreign body reaction was visible.

### Discussion of Examples 1-7

The first objective of this study was to test the ability of HDC to repair annular defects in a needle-punctured rat-tail model. It was found that collagen can preserve the ability of the AF to retain nuclear tissue in the disc space and thereby prevent degenerative changes to the IVD. The potential of collagen gel to induce formation of fibrous tissue that partially restores annular integrity was also observed. The second objective was to evaluate whether adding RF affects the quality of AF repair. It was found that RF improved retention of NP size and disc height for up to 5 or more weeks after needle puncture.

The beneficial effect of RF was dose-dependent. In its absence, collagen gels yielded only a nominal increase in NP size and disc height compared with untreated IVDs. The 0.5-mM RF group retained most of its nuclear tissue according to NP voxel counts and histological cross-sectional area measurements. An average disc height of 84% was maintained. Neither MRI analysis nor histological assessment revealed significant degenerative changes.

There are several potential mechanisms by which cross-linking may have positively influenced AF repair. Chemical cross-linking is known to increase the stiffness and decrease the hydraulic permeability of collagen gels (Cheng et al., "Genipin-Crosslinked Cartilage-Derived Matrix as a Scaffold for Human Adipose-Derived Stem Cell Chondrogenesis," Tissue Eng. Part A 19:484-96 (2013); Stewart et al., "Collagen Cross-Links Reduce Corneal Permeability," Invest. Ophthalmol. Vis. Sci. 50:1606-12 (2009); Tirella et al., "Riboflavin and Collagen: New Crosslinking Methods to Tailor the Stiffness of Hydrogels," Mater. Lett. 74:58-61 (2012). Increased stiffness may help form a more robust barrier for sealing the defect. Decreased hydraulic permeability limits the loss of highly hydrated NP material through the defect (Borde et al., "Repair of Defects in the Rat Tail Annulus Fibrosus Using Injectable High Density Collagen Gels," Paper presented at: Orthopedic Research Society, Annual Meeting; San Francisco, CA (2012). In addition, cross-linking may enhance collagen adhesion to the AF tissue as has been shown with other biopolymer gels (Schek et al., "Genipin-Crosslinked Fibrin Hydrogels as a Potential Adhesive to Augment Intervertebral Disc Annulus Repair," Eur. Cell. Mater. 21:373-83 (2011). This prevents detachment of the implanted material from the annulus and subsequent loss of NP tissue.

A notable histological finding was the formation of a fibrous cap bridging the outer portion of the annular defect in both cross-linked groups. Collagen gel showed varying degrees of host fibroblast infiltration with signs of tissue reorganization, which could explain the formation of this fibrous tissue. The mechanism of host cell invasion with subsequent tissue remodeling of collagen gels has already been described in the literature (Guidry et al., "Studies on the Mechanism of Hydrated Collagen Gel Reorganization by Human Skin Fibroblasts," J. Cell. Sci. 79:67-81 (1985); Staskowski et al., "The Histologic Fate of Autologous Collagen Injected Into the Canine Vocal Fold," Otolaryngol Head Neck Surg. 118:187-90 (1998). Staskowski et al. injected type I collagen gel in a canine model and described that secondary fibroblast invasion led to deposition of new host collagen within the gel. According to this study, cellular infiltration progressed more rapidly in a cross-linked collagen preparation, which could explain why fibrous cap formation occurred only in cross-linked groups. Furthermore, cross-linking is known to delay degradation and increase persistence time of collagen scaffolds, which would also support cell migration and remodeling (Harriger et al., "Glutaraldehyde Crosslinking of Collagen Substrates Inhibits Degradation in Skin Substitutes Grafted to Athymic Mice," J. Biomed. Mater. Res. 35:137-45 (1997); Jarman-Smith et al., "Porcine Collagen Crosslinking, Degradation and its Capability for Fibroblast Adhesion and Proliferation," J. Mater. Sci. Mater. Med. 15:925-32 (2004).

RF is already used clinically to cross-link collagen fibers of the cornea to treat keratoconus (Hashemi et al., "Corneal Collagen Cross-Linking with Riboflavin and Ultraviolet Irradiation for Keratoconus: Long-Term Results," Ophthalmology 120:1515-20 (2013), but it has never been applied to repair IVD tissue. RF serves as a photosensitizer to induce production of oxygen radicals, which then induce chemical bonds between collagen fibrils, thereby cross-linking them (Tirella et al., "Riboflavin and Collagen: New Crosslinking Methods to Tailor the Stiffness of Hydrogels," Mater. Lett. 74:58-61 (2012). Several different substances have been used to cross-link biopolymer gels (Schek et al., "Genipin-Crosslinked Fibrin Hydrogels as a Potential Adhesive to Augment Intervertebral Disc Annulus Repair," Eur. Cell. Mater. 21:373-83 (2011); Harriger et al., "Glutaraldehyde Crosslinking of Collagen Substrates Inhibits Degradation in Skin Substitutes Grafted to Athymic Mice," J. Biomed. Mater. Res. 35:137-45 (1997); Jarman-Smith et al., "Porcine Collagen Crosslinking, Degradation and its Capability for Fibroblast Adhesion and Proliferation," J. Mater. Sci. Mater. Med. 15:925-32 (2004). Using RF is advantageous because it cross-links fibers only after activation by blue light *in situ*, allowing collagen gel to remain liquid prior to injection. Injectable biomaterials may prove more technically feasible for use in spinal procedures than rigid biological implants, because they do not require anchoring to the AF or vertebral body. Moreover, only injectable substances can be applied percutaneously.

The rat-tail model proved to be suitable for testing various compositions of collagen gels. An 18-gauge needle creates a large defect comprising almost the entire posterior wall of the AF in the rat-tail disc. This defect is proportionally much larger than defects induced by annulotomy during discectomies or punctures for discographies. High intradiscal pressure combined with the liquid consistency of the NP leads to rapid extrusion of nuclear tissue if left untreated.

Untreated IVDs degenerated more rapidly than described in other studies that used the same model with a percutaneous approach (Keorochana et al., "The Effect of Needle Size Inducing Degeneration in The Rat Caudal Disc: Evaluation Using Radiograph, Magnetic Resonance Imaging, Histology, and Immunohistochemistry," Spine J 10:1014-23 (2010). This may be due to the open approach used in the study, in which the paravertebral muscles are dissected off the AF, creating a large tissue defect.

## Claims

1. A collagen gel composition for use in repairing an annulus by injecting the composition into, around, or near an annulus repair site under conditions to repair an annulus, the collagen gel composition comprising:
collagen gel and
a cross-linking agent;
wherein the cross-linking agent is riboflavin;
wherein the cross-linking agent is included in the composition in an amount sufficient to cause cross-linking of the collagen gel and enable integration of the collagen gel composition with tissue that comes into contact with the collagen gel composition and the collagen is included in the composition at a concentration of 10 to 180 mg/ml.

2. The composition for use according to claim 1, wherein the collagen is type I collagen.

3. The composition for use according to claim 1 further comprising: living cells.

4. The composition for use according to claim 3, wherein the living cells are selected from chondrocytes, fibroblasts, stem cells, and combinations thereof.

5. The composition for use according to claim 1, wherein the composition is injectable.

6. The composition for use according to claim 1 wherein said injecting does not cause damage to surrounding tissue.

7. The composition for use according to claim 6 wherein said integration is with native tissue of the annulus.

## Patentansprüche

1. Kollagengelzusammensetzung zur Verwendung bei der Reparatur eines Ringes durch Injektion der Zusammensetzung in, um oder in der Nähe einer Ringreparaturstelle unter Bedingungen zur Reparatur eines Ringes, die Kollagengelzusammensetzung umfassend:
Kollagengel und
ein Vernetzungsmittel;
wobei das Vernetzungsmittel Riboflavin ist;
wobei das Vernetzungsmittel in der Zusammensetzung in einer Menge eingeschlossen ist, die ausreicht, um eine Vernetzung des Kollagengels zu bewirken und eine Integration der Kollagengelzusammensetzung mit Gewebe zu ermöglichen, das mit der Kollagengelzusammensetzung in Kontakt kommt, und das Kollagen in der Zusammensetzung in einer Konzentration von 10 bis 180 mg/ml eingeschlossen ist.

2. Zusammensetzung zur Verwendung nach Anspruch 1, wobei es sich bei dem Kollagen um Typ-I-Kollagen handelt.

3. Zusammensetzung zur Verwendung nach Anspruch 1, ferner umfassend:
lebende Zellen.

4. Zusammensetzung zur Verwendung nach Anspruch 3, wobei die lebenden Zellen ausgewählt sind aus Chondrozyten, Fibroblasten, Stammzellen und Kombinationen davon.

5. Zusammensetzung zur Verwendung nach Anspruch 1, wobei die Zusammensetzung injizierbar ist.

6. Zusammensetzung zur Verwendung nach Anspruch 1, wobei die Injektion keine Schädigung des umgebenden Gewebes verursacht.

7. Zusammensetzung zur Verwendung nach Anspruch 6, wobei die Integration mit dem nativen Gewebe des Rings erfolgt.

## Revendications

1. Composition de gel de collagène destinée à être utilisée pour réparer un espace annulaire par l'injection de la composition dans, autour, ou près d'un site de réparation d'espace annulaire dans des conditions pour réparer un espace annulaire, la composition de gel de collagène comprenant :
un gel de collagène et
un agent de réticulation ;
dans laquelle l'agent de réticulation est de la riboflavine ;
dans laquelle l'agent de réticulation est inclus dans la composition en une quantité suffisante pour provoquer une réticulation du gel de collagène et permettre l'intégration de la composition de gel de collagène avec un tissu qui entre en contact avec la composition de gel de collagène et le collagène est inclus dans la composition selon une concentration de 10 à 180 mg/ml.

2. Composition destinée à être utilisée selon la revendication 1, dans laquelle le collagène est du collagène de type I.

3. Composition destinée à être utilisée selon la revendication 1 comprenant en outre des cellules vivantes.

4. Composition destinée à être utilisée selon la revendication 3, dans laquelle les cellules vivantes sont sélectionnées parmi des chondrocytes, des fibroblastes, des cellules souches, et des combinaisons de celles-ci.

5. Composition destinée à être utilisée selon la revendication 1, dans laquelle la composition est injectable.

6. Composition destinée à être utilisée selon la revendication 1 dans laquelle ladite injection ne provoque pas de dommages aux tissus environnants.

7. Composition destinée à être utilisée selon la revendication 6 dans laquelle ladite intégration se fait avec un tissu natif de l'espace annulaire.
